# EUROPEAN PATENT APPLICATION

(11) **EP 3 805 372 A1**
(43) Date of publication of application: **14.04.2021**
(21) Application number: 19815295.1
(22) Date of filing: 06.06.2019
(51) Int. Cl.: C12N 5/079, C12Q 1/02

(54) **METHOD FOR PRODUCING HUMAN A1 ASTROCYTES, HUMAN A1 ASTROCYTES, AND METHOD FOR EVALUATING TEST SUBSTANCE**

(30) Priority: 06.06.2018 JP 2018108618
(71) Applicant: FUJIFILM Corporation, Tokyo 106-8620 (JP)
(72) Inventor: KOBAYASHI Hayato, Ashigarakami-gun, Kanagawa 258-8577 (JP); ENDOH Setsu, Ashigarakami-gun, Kanagawa 258-8577 (JP); NABETANI Akira, Ashigarakami-gun, Kanagawa 258-8577 (JP); SHIN Wigyon, Ashigarakami-gun, Kanagawa 258-8577 (JP)
(74) Representative: Klunker IP Patentanwälte PartG mbB
(86) International application number: PCT/JP2019/022545
(87) International publication number: WO 2019/235576

(57) **Abstract**

The present invention provides a method for producing human A1 astrocytes, which includes inducing human A1 astrocytes from human astrocytes other than human A1 astrocytes; human A1 astrocytes obtained by the method for producing human A1 astrocytes; and a method for evaluating a test substance, which uses the human A1 astrocytes described above. According to the present invention, there is provided the method for producing human A1 astrocytes, which includes a step a of culturing human astrocytes other than human A1 astrocytes in the presence of TNFα and IFNγ.

SEQUENCE LISTING
<110>  FUJIFILM Corporation
<120>  Method for producing human A1 astrocyte, human A1 astrocyte, and
method for screening test substance
<130>\201@18F01625W1
<160>  15
<170>  PatentIn version 3.5
<210>  1
<211>  20
<212>  DNA
<213> Artificial Sequence
<220>
<223> Description of Artificial Sequence: oligonucleotide
<400>  1
tttcaccctg gaactggaag                                                20
<210>  2
<211>  20
<212>  DNA
<213> Artificial Sequence
<220>
<223> Description of Artificial Sequence: oligonucleotide
<400>  2
gacgaagcac ttcctcttgg                                                20
<210>  3
<211>  20
<212>  DNA
<213> Artificial Sequence
<220>
<223> Description of Artificial Sequence: oligonucleotide
<400>  3
ccacgtgttg agatcattgc                                                20
<210>  4
<211>  20
<212>  DNA
<213> Artificial Sequence
<220>
<223> Description of Artificial Sequence: oligonucleotide
<400>  4
cctctgtgtg gtccatcctt                                                20
<210>  5
<211>  20
<212>  DNA
<213> Artificial Sequence
<220>
<223> Description of Artificial Sequence: oligonucleotide
<400>  5
gtcagtggtg gacctgacct                                                20
<210>  6
<211>  20
<212>  DNA
<213> Artificial Sequence
<220>
<223> Description of Artificial Sequence: oligonucleotide
<400>  6
tgctgtagcc aaattcgttg                                               20
<210>  7
<211>  25
<212>  DNA
<213> Artificial Sequence
<220>
<223> Description of Artificial Sequence: oligonucleotide
<400>  7
cacagcggca cagttcatca cggca                                         25

<210>  8
<211>  23
<212>  DNA
<213> Artificial Sequence
<220>
<223> Description of Artificial Sequence: oligonucleotide
<400>  8
gcctattaca acctggagga aag                                          23
<210>  9
<211>  23
<212>  DNA
<213> Artificial Sequence
<220>
<223> Description of Artificial Sequence: oligonucleotide
<400>  9
gtgaccttgt catccgactt ttg                                          23
<210>  10
<211>  31
<212>  DNA
<213> Artificial Sequence
<220>
<223> Description of Artificial Sequence: oligonucleotide
<400>  10
tctgactcta agtggcattc aaggagtacc t                                 31
<210>  11
<211>  22
<212>  DNA
<213> Artificial Sequence
<220>
<223> Description of Artificial Sequence: oligonucleotide
<400>  11
gccattctga tttgctgcct ta                                           22
<210>  12
<211>  25
<212>  DNA
<213> Artificial Sequence
<220>
<223> Description of Artificial Sequence: oligonucleotide
<400>  12
acaggttgat tactaatgct gatgc                                        25
<210>  13
<211>  27
<212>  DNA
<213> Artificial Sequence
<220>
<223> Description of Artificial Sequence: oligonucleotide
<400>  13
catcagcaat gcctcctgca ccaccaa                                      27
<210>  14
<211>  25
<212>  DNA
<213> Artificial Sequence
<220>
<223> Description of Artificial Sequence: oligonucleotide
<400>  14
gaaccatgag aagtatgaca acagc                                       25
<210>  15
<211>  18
<212>  DNA
<213> Artificial Sequence
<220>
<223> Description of Artificial Sequence: oligonucleotide
<400>  15

tgggtggcag tgatggca 18

## Description

### Field of the Invention

The present invention relates to a method for producing human A1 astrocytes and human A1 astrocytes. The present invention further relates to a method for screening a test substance, which uses the produced human A1 astrocytes.

### Description of the Related Art

Astrocytes, which are a type of glial cells that constitutes the brain, play various roles in maintaining homeostasis of the central nervous system, such as supplying nutrients to neurons and forming or removing synapses, and are attracting attention from the viewpoint of elucidating the mechanism of diseases and drug discovery.

It has been known that astrocytes are activated by nerve diseases, aging, or the like. It has recently been reported that there are two types of activated astrocytes, neuropathic A1 astrocytes and neuroprotective A2 astrocytes (Non-Patent Document 1 and Patent Document 1). Human A1 astrocytes are of great value from the viewpoint of drug discovery research, since they can be used for screening drugs that inhibit nerve injury.

Regarding the activation of astrocytes, Non-Patent Document 1 describes that mouse (non-activated) astrocytes are induced to A1 astrocytes by three components of TNFα, IL-1α, and C1q. However, it has been reported that there are species differences between mouse and human astrocytes (Non-Patent Document 2), and it is unclear whether human astrocytes can be induced in the same manner.

Patent Document 2 suggests that inflammatory cytokines such as TNFα and IFNγ are involved in the activation of astrocytes, and describes that mouse (non-activated) astrocytes have been activated with three components of TNFα, IFNγ, and MPTP. However, it was unclear whether the activated astrocytes were induced to A1 astrocytes or A2 astrocytes, and whether similar activation also occurred in human astrocytes.

Non-Patent Document 3 describes that IFNγ and IL-1β are involved in the activation of human astrocytes. However, it was unclear whether the activated astrocytes were induced to A1 astrocytes or A2 astrocytes.

### Prior Art Documents

### Patent Documents

Patent Document 1: US2016/0340648A
Patent Document 2: US2010/0087504A

### Non-Patent Documents

Non-Patent Document 1: Nature, 2017, Vol. 541, pp. 481-487
Non-Patent Document 2: Neuron, 2016, Vol. 89, No. 1, pp. 37-53
Non-Patent Document 3: Glia, 2014, Vol. 62, pp. 999-1013

### SUMMARY OF THE INVENTION

Human A1 astrocytes are of great value from the viewpoint of drug discovery research, and a method for easily obtaining human A1 astrocytes is demanded. As described above, although certain results have been obtained regarding the activation and induction of astrocytes, a method for reliably inducing human A1 astrocytes from human astrocytes has not been found. An object of the present invention is to provide a method for producing human A1 astrocytes, which includes inducing human A1 astrocytes from human astrocytes other than human A1 astrocytes. Another object of the present invention is to provide human A1 astrocytes obtained by the above-described method for producing human A1 astrocytes. Another object of the present invention is to provide a method for evaluating a test substance, which uses the human A1 astrocytes described above.

The inventors of the present invention conducted extensive studies to solve the above problems, and as a result, have found that by culturing human astrocytes other than human A1 astrocytes in the presence of TNFα and IFNγ, the human astrocytes can be induced to human A1 astrocytes. The present invention has been completed based on these findings.

That is, according to the present invention, the following inventions are provided.
<1> A method for producing human A1 astrocytes, comprising: a step a of culturing human astrocytes other than human A1 astrocytes in a presence of TNFα and IFNγ.
<2> The method for producing human A1 astrocytes according to <1>, in which the step a is performed in a further presence of at least one cytokine and/or complement selected from the group consisting of IL-1α, IL-1β, and C1q.
<3> The method for producing human A1 astrocytes according to <1> or <2>, in which the step a is performed in a presence of any one of the followings;
   (1) TNFα, IFNγ, IL-1α, IL-1β
   (2) TNFα, IFNγ, IL-1α, C1q
   (3) TNFα, IFNγ, IL-1β, C1q
   (4) TNFα, IFNγ, IL-1α, IL-1β, C1q, and
   (5) TNFα, IFNγ.
<4> The method for producing human A1 astrocytes according to any one of <1> to <3>, in which the step a includes:
   a step a1 of differentiating human pluripotent stem cells or cells capable of differentiating into human astrocytes into human astrocytes other than human A1 astrocytes; and
   a step a2 of culturing the human astrocytes other than human A1 astrocytes, which are obtained in the step a1, in a presence of TNFα and IFNγ.
<5> The method for producing human A1 astrocytes according to any one of <1> to <4>, in which the human astrocytes other than human A1 astrocytes, which are cultured in the step a, are human A2 astrocytes.
<6> The method for producing human A1 astrocytes according to any one of <1> to <5>, in which the step a is culturing in a serum-free medium.
<7> The method for producing human A1 astrocytes according to any one of <1> to <6>, in which in the step a, a concentration of TNFα in a medium is 0.01 ng/mL to 30 ng/mL.
<8> The method for producing human A1 astrocytes according to any one of <1> to <7>, in which in the step a, a concentration of IFNγ in a medium is 0.1 ng/mL to 20 ng/mL.
<9> The method for producing human A1 astrocytes according to any one of <1> to <8>, in which in the step a, a ratio of a concentration of TNFα in a medium to a concentration of IFNγ in the medium is 100:1 to 1:100.
<10> An isolated human A1 astrocyte obtained by the method for producing human A1 astrocytes according to any one of <1> to <9>.
<11> A method for evaluating a test substance, comprising bringing a test substance into contact with the human A1 astrocytes according to <10>.
<12> The method for evaluating a test substance according to <11>, further comprising evaluating a neuropathic change of the human A1 astrocytes after the bringing of the test substance into contact with the human A1 astrocytes according to <10>.

According to the present invention, human A1 astrocytes can be produced from human astrocytes. The human A1 astrocytes according to an aspect of the present invention and the method for evaluating a test substance according to an aspect of the present invention are useful in drug discovery research and the like.

### BRIEF DESCRIPTION OF THE DRAWINGS

Fig. 1 shows the appearance of human astrocytes after being cultured in the presence of a specific combination of compounds.
Fig. 2 shows the appearance of human astrocytes (induced from astrocyte progenitor cells) after being cultured in the presence of a specific combination of compounds.
Fig. 3 shows the results of quantifying the expression of GBP2 and CXCL10 after culturing human astrocytes in the presence of a specific combination of compounds.
Fig. 4 shows the results of quantifying the expression of C3 after culturing human astrocytes in the presence of a specific combination of compounds.
Fig. 5 shows the result of quantifying the expression of C3 after culturing human astrocytes (induced from astrocyte progenitor cells) in the presence of a specific combination of compounds.
Fig. 6 shows the result of quantifying the expression of C3 after culturing human astrocytes (primary culture) in the presence of a specific combination of compounds.
Fig. 7 shows the appearance of nerve cells (also referred to as neurons) which have been cultured together with untreated human astrocytes or with human astrocytes cultured in the presence of a specific combination of compounds.
Fig. 8 shows the evaluation results of the cell death of nerve cells (also referred to as neurons) which have been cultured together with untreated human astrocytes or with human astrocytes cultured in the presence of a specific combination of compounds.
Fig. 9 shows the appearance of nerve cells and the length of neurites in a case where the nerve cells were cultured together with untreated human astrocytes (induced from astrocyte progenitor cells) or in a case where nerve cells were cultured together with human astrocytes (induced from astrocyte progenitor cells) in the presence of a specific combination of compounds.

### DESCRIPTION OF THE PREFERRED EMBODIMENTS

Hereinafter, the embodiments of the present invention will be described in detail.
TNFα refers to Tumor Necrosis Factor α.
IFNγ refers to Interferon γ.
IL-1α refers to Interleukin-1α.
IL-1β refers to Interleukin-1β.
C1q refers to complement C1q.
MPTP refers to 1-methyl-4-phenyl-1,2,3,6-tetrahydropyridine.
EMP1 refers to Epithelial membrane protein 1.
CD109 refers to Cluster of Differentiation 109.
GBP2 refers to guanylate binding protein 2.
C3 refers to complement molecule C3.
CXCL10 refers to C-X-C motif chemokine 10.
GAPDH refers to glyceraldehyde 3-phosphate dehydrogenase.
HBEGF refers to heparin binding-epidermal growth factor-like growth factor.
WST refers to Water soluble Tetrazolium salts.

The present invention relates to a method for producing human A1 astrocytes, which includes a step a of culturing human astrocytes other than human A1 astrocytes in the presence of TNFα and IFNγ.

Step a is a step of culturing human astrocytes other than human A1 astrocytes in the presence of TNFα and IFNγ, or in the presence of at least one cytokine and/or complement selected from the group consisting of IL-1α, IL-1β, and C1q, in addition to TNFα and IFNγ. The step a is preferably a step of culturing human astrocytes other than human A1 astrocytes in the presence of any one of the followings.
(1) TNFα, IFNγ, IL-1α, IL-1β
(2) TNFα, IFNγ, IL-1α, C1q
(3) TNFα, IFNγ, IL-1β, C1q
(4) TNFα, IFNγ, IL-1α, IL-1β, C1q, and
(5) TNFα, IFNγ.

The step a is particularly preferably a step of culturing human astrocytes other than human A1 astrocytes in the presence of any one of the followings.
(4) TNFα, IFNγ, IL-1α, IL-1β, C1q, and
(5) TNFα, IFNγ.

The step a is preferably a step of culturing human astrocytes other than human A1 astrocytes in the absence of MPTP.

### <Human astrocytes>

"Human astrocytes" are astrocytes of human.

Examples of the method for obtaining the "human astrocytes other than human A1 astrocytes" used in the present invention include separating from the cerebral cortex, spinal cord, or the like, which is surgically obtained from a patient, inducing from cells capable of differentiating into human astrocytes, and inducing from human pluripotent stem cells. Any of these human astrocytes can be used as the "human astrocytes other than human A1 astrocytes" in the method according to the embodiment of the present invention.

As the "human astrocytes other than human A1 astrocytes" used in the present invention, human astrocytes induced from human pluripotent stem cells and human astrocytes induced from cells capable of differentiating into human astrocytes can be preferably used.

The human astrocytes other than human A1 astrocytes can be used in the method according to the embodiment of the present invention in an isolated state or in a state of being mixed with other cells. Further, it can also be considered that the induction from pluripotent stem cells to human astrocytes and the induction from human astrocytes to human A1 astrocytes are performed consecutively. In this case, the step a includes a step a1 of differentiating human pluripotent stem cells or cells capable of differentiating into human astrocytes into human astrocytes other than human A1 astrocytes; and a step a2 of culturing the human astrocytes other than human A1 astrocytes, which are obtained in the step a1, in a presence of TNFα and IFNγ.

As the human pluripotent stem cells, human induced pluripotent stem (iPS) cells, human embryonic stem (ES) cells, and human mesenchymal stem cells can be mentioned, which are not particularly limited.

As the cells capable of differentiating into human astrocytes, neural stem cells, glial neural progenitor cells, glial progenitor cells, astrocyte progenitor cells, and fibroblasts can be mentioned, which are not particularly limited.

### <Activated human astrocytes>

"Activated human astrocytes" are human astrocytes activated by the influence of neurological diseases or the like. As the activated human astrocytes, human A1 astrocytes that are characterized by neuropathic properties and neuroprotective human A2 astrocytes are known. The activated human astrocytes are also included in the human astrocytes according to the embodiment of the present invention.

### <Human A1 astrocytes>

The "Human A1 astrocytes" is a type of activated human astrocytes.

The human A1 astrocytes produced by the method according to the embodiment of the present invention can be detected, confirmed, and separated by utilizing, for example, morphological changes of cells, characteristic properties of human A1 astrocytes, and specific markers.

The human A1 astrocytes have the characteristic appearance of hypertrophy. Accordingly, the human A1 astrocytes can be detected by observation with a microscope.

As the specific markers for human A1 astrocytes, GBP2, CXCL10, and C3 are mentioned but are not limited thereto.

A immunological method (detection with an antibody) can be used to detect the specific markers, but the detection may be carried out by quantifying the amount of mRNA of the protein molecule in a case where the specific markers are proteins.

Human A1 astrocytes exhibit neuronal cytotoxicity. Accordingly, the human A1 astrocytes can be detected by co-culturing with nerve cells and utilizing the cytotoxicity on the nerve cells.

### <Human A2 astrocytes>

The "human A2 astrocytes" is a type of activated human astrocytes.

A2 astrocytes are up-regulated in various neurotrophic factors and act protectively on nerve cells. As the specific markers of the A2 astrocytes, EMP1 and CD109 are mentioned but not limited thereto.

The human A2 astrocytes have a star-shaped appearance.

In the present invention, the human A2 astrocytes can be used as the human astrocytes other than human A1 astrocytes, which are cultured in the step a.

### <TNFα>

TNFα is a kind of cytokine and is a substance widely involved in biophylaxis and activation of the immune mechanism during inflammation. The means for achieving the culture conditions in "in the presence of TNFα" is not particularly limited, and examples thereof include adding TNFα to a medium, co-culturing with cells that produce TNFα, and adding the culture supernatant of cells that produce TNFα.

The concentration of TNFα may be appropriately determined and is not particularly limited, but for example, TNFα can be used in the range of 0.01 ng/mL to 30 ng/mL and preferably in the range of 0.5 ng/mL to 10 ng/mL.

### <IFNγ>

IFNγ is a cytokine that controls the induction of cell-mediated immunity. The means for achieving the culture conditions "in the presence of IFNγ" is not particularly limited, and examples thereof include adding IFNγ to a medium, co-culturing with cells that produce IFNγ, and adding the culture supernatant of cells that produce IFNγ.

The concentration of IFNγ may be appropriately determined and is not particularly limited, but for example, IFNγ can be used in the range of 0.1 ng/mL to 20 ng/mL and preferably int the range of 0.5 ng/mL to 10 ng/mL.

The ratio of the concentration of TNFα to the concentration of IFNγ in the medium is not particularly limited, but is preferably 100:1 to 1:100, more preferably 10:1 to 1:10, and still more preferably 5:1 to 1:5, and particularly preferably 3:1 to 1:1.

### <IL-1α>

IL-la is a kind of interleukin that is a cytokine secreted by leukocytes. The means for achieving the culture conditions "in the presence of IL-1α" is not particularly limited, and examples thereof include, adding IL-1α to a medium, co-culturing with cells that produce IL-1α, and adding the culture supernatant of cells that produce IL-1α.

The concentration of IL-1α may be appropriately determined and is not particularly limited, but for example, IL-1α can be used in the range of 1 pg/mL to 5 ng/mL and preferably in the range of 0.05 ng/mL to 3 ng/mL.

### <IL-1β>

IL-1β is a kind of interleukin that is a cytokine secreted by leukocytes. The means for achieving the culture conditions "in the presence of IL-1β" is not particularly limited, and examples thereof include, adding IL-1β to a medium, co-culturing with cells that produce IL-1β, and adding the culture supernatant of cells that produce IL-1β.

The concentration of IL-1β may be appropriately determined and is not particularly limited, but for example, IL-1β can be used in the range of 1 pg/mL to 10 ng/mL and preferably in the range of 0.5 ng/mL to 5 ng/mL.

### <C1q>

C1q is one of complements. C1q is a factor that is the origin of the classical pathway, which is one of the complement activation pathways. The means for achieving the culture conditions "in the presence of C1q" is not particularly limited, and examples thereof include adding C1q to a medium, co-culturing with cells that produce C1q, and adding the culture supernatant of cells that produce C1q.

The concentration of C1q may be appropriately determined and is not particularly limited, but for example, C1q can be used in the range of 10 ng/mL to 10 µg/mL and preferably in the range of 50 ng/mL to 300 ng/mL.

### <Culture of human astrocytes>

The culture of human astrocytes in the present invention may be carried out in the presence of the above-described various cytokines and/or complement by selecting a medium, temperature, and other conditions depending on the origin and state of the human astrocytes to be used. The medium may contain components in addition to the above-described various cytokines and/or complement as long as the components do not interfere with the culture of human astrocytes in the present invention. A medium can be selected from the known media and commercially available media. For example, suitable components (serum, protein, amino acid, sugar, vitamin, fatty acid, antibiotics, and the like) are added to a general medium such as minimum essential medium (MEM), Dulbecco's modified Eagle medium (DMEM), DMEM/F12, or a medium obtained by modifying these media, for using in cell culture. A medium not containing serum (serum-free medium) is preferably used as the medium.

As the culture conditions, general cell culture conditions may be selected. For example, conditions of 37°C and 5% CO₂.are mentioned. During the culture, it is preferable to change the medium at appropriate intervals (preferably once a day to 7 days and more preferably once every 2 days to 3 days). In a case where the method according to the embodiment of the present invention is carried out using human astrocytes as a material, human A1 astrocytes appear in one day to one week under the conditions of 37°C and 5% CO₂.

For culturing somatic cells, cell culture vessels such as plates, dishes, cell culture inserts, cell culture flasks, and cell culture bags can be used. As the cell culture bag, a cell culture bag having gas permeability is suitable. Larger culture tanks may be used in a case where a large number of cells are required. The culturing can be performed in either an open system or a closed system.

In the method according to the embodiment of the present invention, human A1 astrocytes can be produced by culturing human astrocytes in the medium containing the above-described various cytokines and/or complement.

<Human A1 astrocytes and method for evaluating test substance using human A1 astrocytes>

The present invention provides an isolated human A1 astrocyte obtained by the method for producing human A1 astrocytes according to the embodiment of the present invention.

The human A1 astrocytes produced by the method according to the embodiment of the present invention can also be used for screening drug candidate compounds that act on human A1 astrocytes and for evaluating the safety of drug candidate compounds.

The present invention provides a method for evaluating a test substance, which includes bringing a test substance into contact with the human A1 astrocytes according to the embodiment of the present invention. For example, the test substance may be evaluated by evaluating change in neuronal cytotoxicity of the human A1 astrocytes after bringing the test substance into contact with the human A1 astrocytes. The change in neuronal cytotoxicity of human A1 astrocytes can be evaluated, for example, by co-culturing human A1 astrocytes with nerve cells according to the method described in Examples described later and utilizing the cytotoxicity on the nerve cells.

The present invention will be more specifically described with reference to Examples, but the present invention is not limited to the scope of Examples.

### Examples

### Test Example 1: Induction from human astrocytes to human A1 astrocytes

### (1) Human astrocytes

### (1-1) Astrocytes derived from human iPS cells

As human astrocytes, iCell (registered trademark) Astrocytes (CDI, ASC-100-020-001-PT), which are astrocytes derived from human iPS cells, were purchased from FUJIFILM Cellular Dynamics, Inc. and used. From the appearance, it was confirmed that iCell (registered trademark) Astrocytes were human astrocytes other than human A1 astrocytes.

### (1-2) Astrocytes induced from astrocyte progenitor cells

In addition, as human astrocytes, commercially available human astrocyte progenitor cells (AX0083, manufactured by Axol Bioscience Ltd.) were also induced under the conditions described in the method attached to the kit and used. From the appearance, it was confirmed that the induced human astrocytes were human astrocytes other than human A1 astrocytes.

### (1-3) Human-derived primary cultured astrocytes

Further, commercially available primary cultured human astrocytes (CC-2565, manufactured by Lonza) were also used.

### (2) Induction to human A1 astrocytes

Induction to human A1 astrocytes was performed by an induction method 1 in Examples 1 to 4 and Comparative Examples 1 to 4, and by an induction method 2 in Example 5. An induction method 3 was used in Examples 6 and 7.

### Induction method 1

Matrigel (registered trademark) basement membrane matrix (356234, manufactured by Corning Inc.) diluted 120 folds with DMEM (11960-044, manufactured by Thermo Fisher Scientific, Inc.) was added to a 6-well plate at 1.5 mL/well and allowed to be left at 4°C. After 24 hours, the medium was replaced with the following serum-free medium for use.

### Composition of serum-free medium

Neurobasal (registered trademark) medium (50%, 21103049, manufactured by Thermo Fisher Scientific, Inc.)
DMEM (50%)
Penicillin/Streptomycin (× 100) (1 ×, 15140-122, manufactured by Thermo Fisher Scientific, Inc.)
Sodium pyruvate (1 mM, 11360-070, manufactured by Thermo Fisher Scientific, Inc.)
L-glutamine (292 µg/mL, 25030-081, manufactured by Thermo Fisher Scientific, Inc.)
N2 Supplement with Transferrin (Apo) (1 ×, 141-09041, manufactured by Wako)
N-acetyl system (5 µg/mL, A8199, manufactured by Sigma-Aldrich Co. LLC) HBEGF (5 ng/mL, 100-47, manufactured by PeproTech Inc.)

iCell (registered trademark) astrocytes were suspended in a serum-free medium, seeded on a 6-well plate at a density of 30 × 10⁴ cells/well and cultured under the conditions of 37°C and 5% CO₂ for 24 hours.

The medium was replaced with a serum-free medium to which two to five of the compounds (TNFα, 8902SC, manufactured by Cell Signaling Technology Inc.), IFNγ (285-IF, manufactured by R&D Systems Inc.), IL-la (SRP3310, manufactured by Sigma-Aldrich Co. LLC), IL-1β (201-LB, manufactured by R&D Systems Inc.), and C1q (MBS143105, manufactured by MyBioSource Inc.)) described in Table 1 were added, and the astrocytes were cultured under the conditions of 37°C and 5% CO₂ for one day.

### Induction method 2

Matrigel (registered trademark) basement membrane matrix (356234, manufactured by Corning Inc.) diluted 120 folds with DMEM (11960-044, manufactured by Thermo Fisher Scientific, Inc.) was added to a 96-well plate at 65 µL/well and allowed to be left at 4°C. After 24 hours, the medium was replaced with the same serum-free medium as the serum-free medium used in the Induction method 1 and used.

iCell (registered trademark) astrocytes were suspended in a serum-free medium, seeded on a 96-well plate at a density of 3 × 10⁴ cells/well, and cultured under the conditions of 37°C and 5% CO₂ for 24 hours.

The medium was replaced with a serum-free medium to which two of the compounds described in Table 1 were added, and the astrocytes were cultured under the conditions of 37°C and 5% CO₂ for one day.

### Induction method 3

Matrigel (registered trademark) basement membrane matrix (356234, manufactured by Corning Inc.) diluted 120 folds with DMEM (11960-044, manufactured by Thermo Fisher Scientific, Inc.) was added to a 96-well plate at 65 µL/well, allowed to be left at 4°C, and used after 24 hours.

In Example 6 and in Example 7 respectively, astrocytes induced from commercially available human astrocyte progenitor cells and primary cultured human astrocytes were seeded on a 96-well plate at a density of 3 × 10⁴ cells/well and cultured under the conditions of 37°C and 5% CO₂ for 24 hours.

The medium was replaced with a medium to which two of the compounds described in Table 1 were added, and the astrocytes were cultured under the conditions of 37°C and 5% CO₂ for one day.

### Immunostaining

In Example 6, staining of GFAP, which is a marker for astrocytes, was performed by immunostaining. 80% ethanol (100 µL/well) was added to astrocytes induced from human astrocyte progenitor cells induced by the induction method 3, and the astrocytes were fixed at -30°C for 24 hours. After washing 3 times with PBS, the astrocytes were blocked by adding a PBS solution (1% BSA) containing 1% BSA, and treated with a primary antibody (MAB3402, manufactured by Merck Millipore) diluted 3,000 folds with 1% BSA at 4°C for 24 hours. After washing 3 times with PBS, the astrocytes were treated with a secondary antibody (A11005, manufactured by ThermoFisher Scientific Inc.) diluted 1,000 folds with 1% BSA at room temperature for 1 hour. After washing 3 times with PBS, images were acquired by photographing with IncuCyte S3 (4647, manufactured by Essen BioScience).

**[Table 1] (The unit for the numerical values in Table is ng/mL)**

| | TNFα | IFNγ | IL-1α | IL-1β | C1q |
|---|---|---|---|---|---|
| Example 1 | 7.5 | 5 | 0.75 | 2.5 | 100 |
| Example 2 | 7.5 | 5 | 0.75 | 2.5 | |
| Example 3 | 7.5 | 5 | 0.75 | | 100 |
| Example 4 | 7.5 | 5 | | 2.5 | 100 |
| Example 5 | 7.5 | 5 | | | |
| Example 6 | 7.5 | 5 | | | |
| Example 7 | 7.5 | 5 | | | |
| Comparative Example 1 | 7.5 | | 0.75 | 2.5 | 100 |
| Comparative Example 2 | | 5 | 0.75 | 2.5 | 100 |
| Comparative Example 3 | | 5 | | 2.5 | |
| Comparative Example 4 | 7.5 | | 0.75 | | 100 |

### (3) Evaluation of cell morphology

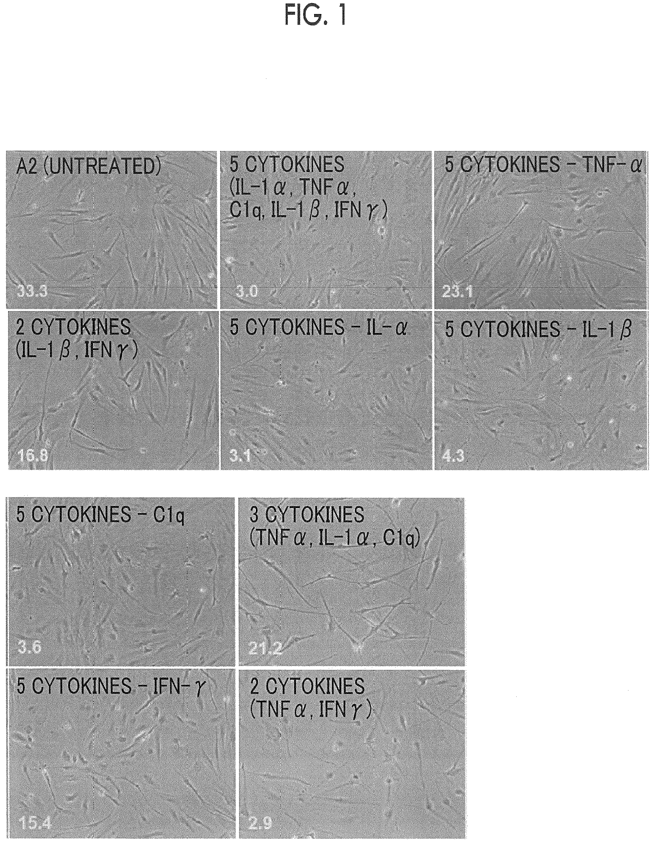

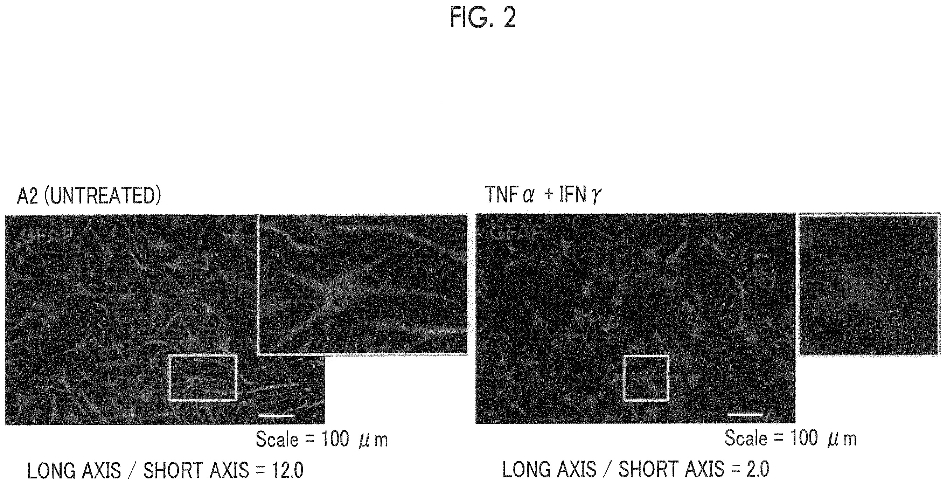

The morphology of the cells after culture was observed with a microscope or a photographed image. The results are shown in Fig. 1 and Fig. 2. In addition, the results of evaluating the morphology from Figs. 1 and 2 are shown in Table below. The morphology was determined by selecting 10 cells/field from typical cells and comparing the long axis with the short axis. Cells with a ratio of long axis/short axis of 10 or less were defined as hypertrophic, cells with a ratio of long axis/short axis of more than 10 and having fibrous shape was defined as fibrous, and cells with a ratio of long axis/short axis of more than 10 and having a plurality of protrusions were defined as star-shaped. The numerical values of the evaluation results of the ratio of long axis/short axis are shown in Figs. 1 and 2. The magnified view in the frame of Fig. 2 shows a typical star-shaped (left side of the figure) and hypertrophic (right side of the figure) cell.

**[Table 2]**

| | |
|---|---|
| | Cell morphology |
| Example 1 | All hypertrophic |
| Example 2 | All hypertrophic |
| Example 3 | All hypertrophic |
| Example 4 | All hypertrophic |
| Example 5 | All hypertrophic |
| Example 6 | All hypertrophic |
| Comparative Example 1 | Fibrous, star-shaped |
| Comparative Example 2 | Fibrous, star-shaped |
| Comparative Example 3 | Fibrous, star-shaped |
| Comparative Example 4 | Fibrous, star-shaped |

From Fig. 1, Fig. 2, and Table 2, it can be seen that in Examples 1 to 6, substantially all the cells exhibited the hypertrophic morphology characteristic of human A1 astrocytes. On the other hand, in Comparative Examples 1 to 4, it can be seen that there are no or very few cells that exhibit a hypertrophic morphology.

### (4) Evaluation of mRNA expression level

The amount of genes expressed by the cells after culturing was measured by quantitative polymerase chain reaction (PCR). The quantitative PCR was carried out by a quantitative PCR method 1 in Examples 1 to 4 and Comparative Examples 1 to 4, and by a quantitative PCR method 2 in Examples 5 to 7.

### Quantitative PCR method 1

Total RNA was extracted from cells one day after induction. RNeasy (registered trademark) mini kit (74104, manufactured by QIAGEN) was used for the extraction of total RNA according to the attached manual. PrimeScript (registered trademark) RT reagent Kit with gDNA Eraser (Perfect Real Time) (RR047A, manufactured by Takara Bio Inc.) was used for reverse transcription of total RNA according to the attached manual. For quantitative PCR, TB Green Premix Ex Taq II (Tli RNase H Plus) (RR820B, manufactured by Takara Bio Inc.) was used according to the attached protocol. Mx3005P (manufactured by Stratagene) was used and, as the cycle program, one cycle of 95°C for 30 seconds and 45 cycles of 95°C for 5 seconds and 60°C for 30 seconds were performed. The sequences of the primers are shown below.

### GBP2

Forward primer: tttcaccctggaactggaag (SEQ ID NO: 1)
Reverse primer: gacgaagcacttcctcttgg (SEQ ID NO: 2)

### CXCL10

Forward primer: ccacgtgttgagatcattgc (SEQ ID NO: 3)
Reverse primer: cctctgtgtggtccatcctt (SEQ ID NO: 4)

### GAPDH

Forward primer: gtcagtggtggacctgacct (SEQ ID NO: 5)
Reverse primer: tgctgtagccaaattcgttg (SEQ ID NO: 6)

### Quantitative PCR method 2

mRNA was quantified from cells one day after induction using FastLane cell Multiplex NR Kit (216513, manufactured by QIAGEN) according to the attached manual. The primers and the fluorescent probes were synthesized by the Dual Labeled Probe design set (manufactured by Takara Bio Inc.). CFX384 Touch real-time PCR analysis system (manufactured by Bio-Rad Laboratories Inc.) was used and, as the cycle program, one cycle of 50°C for 20 minutes, one cycle of 95°C for 15 minutes, and 45 cycles of 94°C for 45 seconds and 60°C for 75 seconds were performed. The sequences of the primer and the fluorescent probe are shown below.
C3 probe: 5'-(FAM)CACAGCGGCACAGTTCATCACGGCA(BHQ1)-3' (SEQ ID NO: 7)
Forward primer: GCCTATTACAACCTGGAGGAAAG (SEQ ID NO: 8)
Reverse primer: GTGACCTTGTCATCCGACTTTTG (SEQ ID NO: 9)
CXCL10 probe: 5'-(Cyanine5)TCTGACTCTAAGTGGCATTCAAGGAGTACCT(BHQ1)-3' (SEQ ID NO: 10)
Forward primer: GCCATTCTGATTTGCTGCCTTA (SEQ ID NO: 11)
Reverse primer: ACAGGTTGATTACTAATGCTGATGC (SEQ ID NO: 12)
GAPDH probe: 5'-(HEX)CATCAGCAATGCCTCCTGCACCACCAA(BHQ1)-3' (SEQ ID NO: 13)
Forward primer: GAACCATGAGAAGTATGACAACAGC (SEQ ID NO: 14)
Reverse primer: TGGGTGGCAGTGATGGCA (SEQ ID NO: 15)

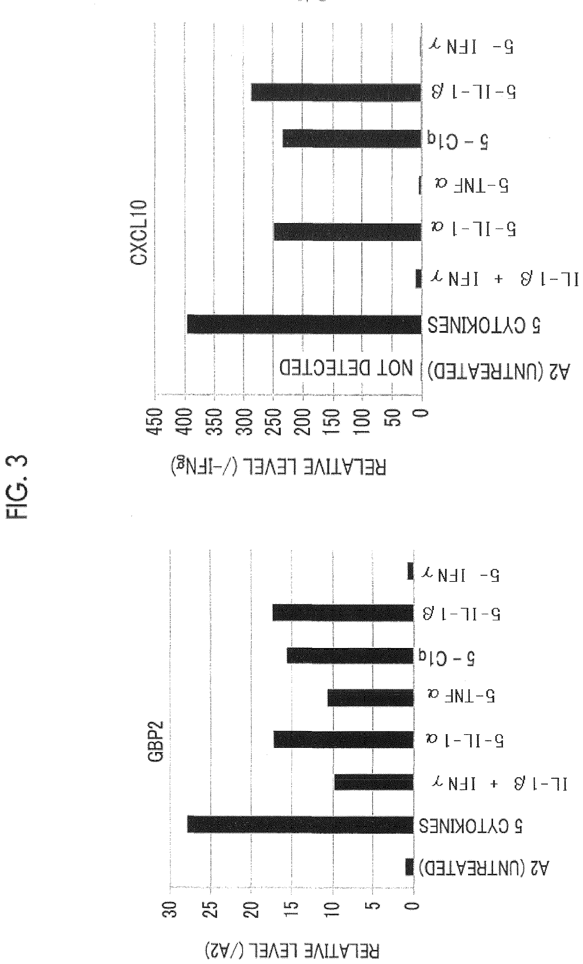

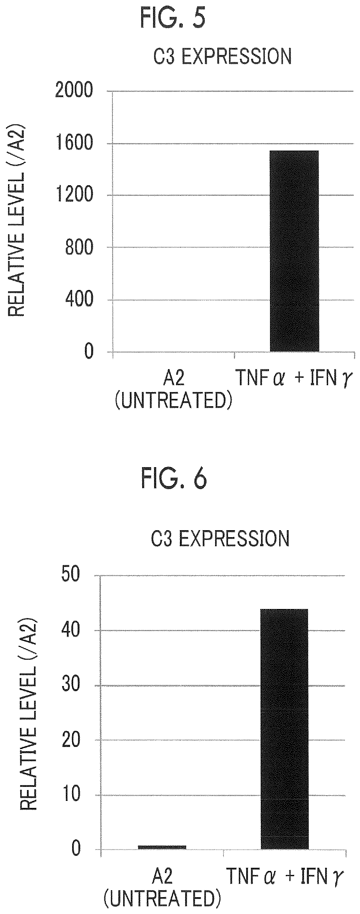

The expression level of mRNA was quantified by the ΔΔCt method (comparative Ct method) using GAPDH as the internal standard. The results are shown in Fig. 3, Fig, 4, Fig. 5, and Fig.6.

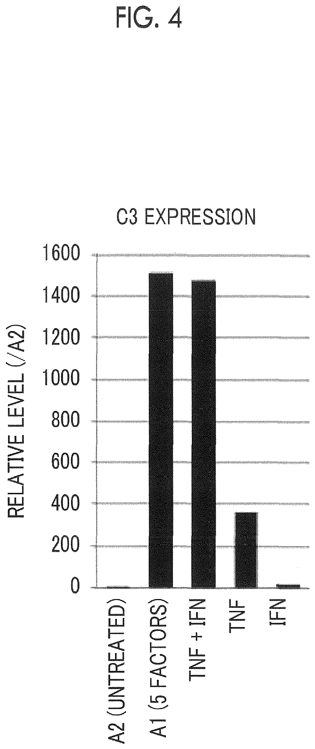

From Fig. 3, it can be seen that in Examples 1 to 4, both GBP2 and CXCL10 are sufficiently expressed. On the other hand, in Comparative Examples 1 to 4, almost no GBP2 and CXCL10 are expressed, or even in a case of being expressed, the amount thereof is small compared to Examples. From Fig. 4, it can be seen that the expression of C3 is increased by stimulation in Examples 1 and 5. From Fig.5 and Fig. 6, it can be seen that the expression of C3 is increased by stimulation in Examples 6 and 7.

### Test Example 2: Co-culture of human A1 astrocytes and nerve cells

### (1) Seeding of human astrocytes on cell culture insert

As human astrocytes, iCell (registered trademark) astrocytes were used as in Test Example 1.

Matrigel (registered trademark) diluted 120 folds with DMEM was added to cell culture insert (353104, manufactured by Corning Inc.) at 100 µL/well and allowed to be left at 4°C. After 24 hours, the medium was replaced with the same serum-free medium as the serum-free medium used in the Induction method 1. iCell (registered trademark) astrocytes were suspended in a serum-free medium, seeded on a cell culture insert at a density of 5 × 10⁴ cells/well, and cultured under the conditions of 37°C and 5% CO₂.

After 24 hours, the medium was replaced with a serum-free medium or a serum-free medium to which IL-1α (0.75 ng/mL), TNFα (7.5 ng/mL), C1q (100 ng/mL), IL-1β (2.5 ng/mL), and IFNγ (5 ng/mL) were added.

### (2) Seeding of nerve cells on culture plate

Poly-L-Lysine solution (P4707, manufactured by Sigma-Aldrich Co. LLC) diluted 10 folds with phosphate buffered solution (PBS) was added to a 24-well plate at 500 µL/well and allowed to be left at room temperature. After 24 hours, iMatrix-511 (892012, manufactured by Nippi. Inc.) diluted 166 folds with PBS was added to a 24-well plate at 500 µL/well, incubated at 37°C for 1 hour, and then replaced with a serum-free medium.

According to the method disclosed in WO2014/148646A1, Neurogenin2 was forcibly expressed to differentiate iPS cells into nerve cells. The obtained nerve cells were suspended in a serum-free medium, seeded on a 24-well plate at a density of 10 × 10⁴ cells/well, and cultured under the conditions of 37°C and 5% CO₂.

### (3) Co-culture with nerve cells

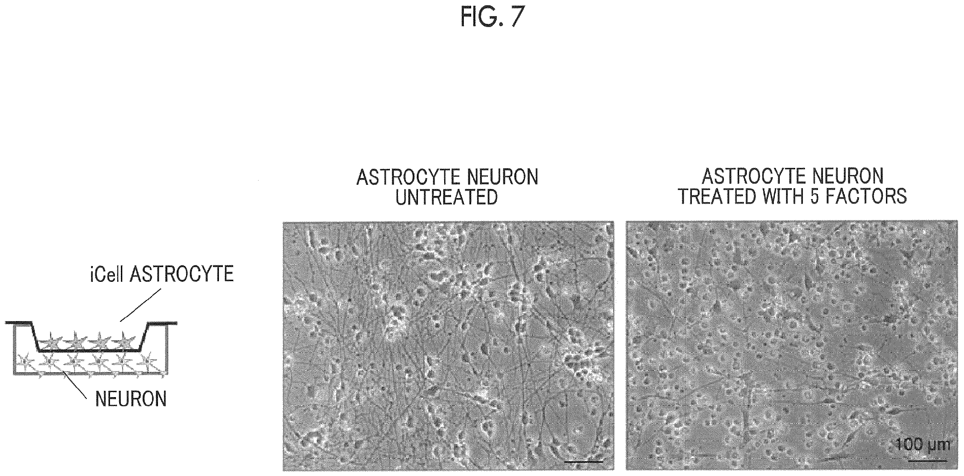

The culture medium in the cell culture insert on which human astrocytes were seeded and the culture medium in the culture plate on which nerve cells were seeded were replaced with a serum-free medium or a serum-free medium containing the five compounds, and the combined co-culture was started. As a control, a single culture of nerve cells was also started at the same time. After culturing under the conditions of 37°C and 5% CO₂ for 5 days, the appearance of nerve cells was observed. The results are shown in Fig. 7.

It can be seen that human astrocytes replaced with serum-free medium act protectively on nerve cells and co-cultured nerve cells have a larger number of viable cells and firm neurites as compared with nerve cells cultured singly. On the other hand, it can be seen that human astrocytes replaced with the serum-free medium containing the five compounds exhibited neurotoxicity, and the co-cultured nerve cells have many dead cells, as compared with the nerve cells co-cultured with the astrocytes replaced with the serum-free medium, and have a reduced number of viable cells and neurites.

### (4) Quantitative evaluation of nerve cell death

After co-culturing for 5 days, viable cells were quantitatively evaluated by the WST test and the counting of the viable cells. The WST test was evaluated using Cell Counting Kit-8 (CK04, manufactured by Dojindo Molecular Technologies. Inc.). After removing the cell culture insert, 50 µL of Cell Counting Kit solution was added to the culture plate, and color reaction was performed for 1 to 4 hours under the conditions of 37°C and 5% CO₂. Then, 100 µL of the reaction solution was measured for absorbance at 450 nm and 650 nm using a microplate reader, and quantification was performed based on the value obtained by subtracting the value at 650 nm from the value at 450 nm. The number of viable cells was evaluated using Cellstain (registered trademark) CytoRed solution (C410, manufactured by Dojindo Molecular Technologies. Inc.) and IncuCyte (registered trademark) S3 (4647, manufactured by Essen BioScience) using the sample after performing the WST test. 0.5 µL of 1 mmol/L CytoRed solution was added to the culture plate, color reaction was performed for 30 minutes to 2 hours under the conditions of 37°C and 5% CO₂, whereby viable cells were stained. Then, the number of viable cells was counted using IncuCyte (registered trademark) S3.

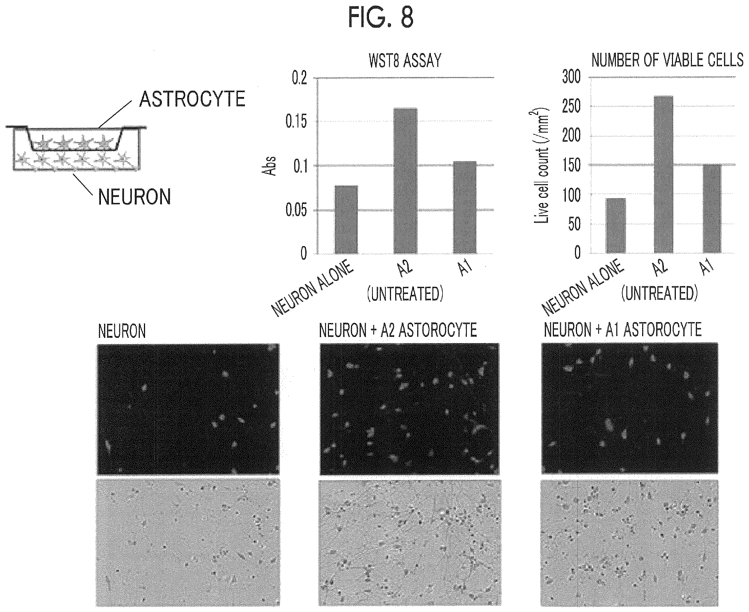

### The results are shown in Fig. 8.

As a result of the WST test and quantification of the number of viable cells, it has been confirmed that the nerve cells co-cultured with the human astrocytes in the replaced serum-free medium have a larger number of viable cells as compared with nerve cells cultured singly. On the other hand, it has been confirmed that the nerve cells co-cultured with the human astrocytes in the replaced serum-free medium containing the five compounds have a reduced number of viable cells as compared with the nerve cells co-cultured with the astrocytes in the replaced serum-free medium.

### Test Example 3: Neuropathic properties of various human astrocytes

### (1) Seeding of astrocytes induced from human astrocyte progenitor cells

Matrigel (registered trademark) basement membrane matrix (356234, manufactured by Corning Inc.) diluted 120 folds with DMEM (11960-044, manufactured by Thermo Fisher Scientific, Inc.) was added to a 96-well plate at 65 µL/well and allowed to be left at 4°C. After 24 hours, astrocytes induced from commercially available human astrocyte progenitor cells were seeded at a density of 5 × 10⁴ cells/well.

The astrocytes were treated with (A) unstimulation, (B) stimulation with TNFα (7.5 ng/mL) and IFNγ (5 ng/mL), or (C) stimulation with TNFα (7.5 ng/mL), IL-la (0.75 ng/mL), and C1q (100 ng/mL), and nerve cells were seeded 48 hours later.

### (2) Seeding of nerve cells on culture plate

According to the method disclosed in WO2014/148646A1, Neurogenin2 was forcibly expressed to differentiate iPS cells into nerve cells. The obtained nerve cells were seeded on the astrocytes induced to A1 type at a density of 3 × 10⁴ cells/well and cultured for 3 days under the conditions of 37°C and 5% CO₂.

### (3) Immunostaining

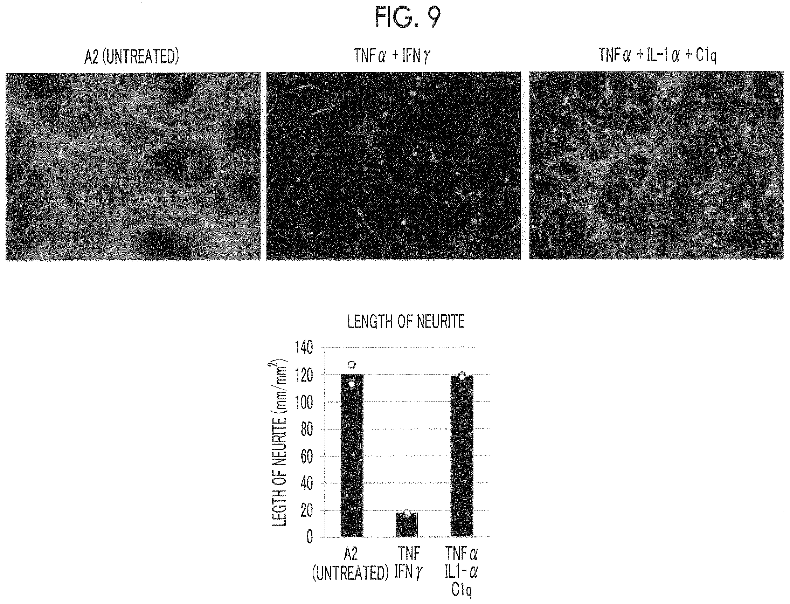

80% ethanol (100 µL/well) was added to the cells cultured for 3 days and fixed at -30°C for 24 hours. After washing 3 times with PBS, blocking was performed by adding 1% BSA, and a primary antibody (MRB-435P, manufactured by Covance Inc.) diluted 2,000 folds with 1% BSA was treated at 4°C for 24 hours. After washing 3 times with PBS, a secondary antibody (ThermoFisher Scientific, A11008) diluted 1,000 folds with 1% BSA was treated at room temperature for 1 hour. After washing 3 times with PBS, images were acquired by photographing with IncuCyte S3 (4647, manufactured by Essen BioScience). The results are shown in Fig. 9.

In a case where nerve cells respectively co-cultured with human astrocytes cells treated with (A) unstimulation, human astrocytes treated with (B) stimulation, and human astrocytes treated with (C) stimulation are compared, it has been confirmed that the nerve cells co-cultured with the astrocytes treated with (B) stimulation has a reduced number of neurites.

### [Sequence list] International application 18F01625W1JP19022545_2.app based on International Patent Cooperation Treaty

## Claims

1. A method for producing human A1 astrocytes, comprising: a step a of culturing human astrocytes other than human A1 astrocytes in a presence of TNFα and IFNγ.

2. The method for producing human A1 astrocytes according to claim 1, wherein the step a is performed in a further presence of at least one cytokine and/or complement selected from the group consisting of IL-1α, IL-1β, and C1q.

3. The method for producing human A1 astrocytes according to claim 1 or 2, wherein the step a is performed in a presence of any one of the followings;
(1) TNFα, IFNγ, IL-1α, IL-1β
(2) TNFα, IFNγ, IL-1α, C1q
(3) TNFα, IFNγ, IL-1β, C1q
(4) TNFα, IFNγ, IL-1α, IL-1β, C1q, and,
(5) TNFα, IFNγ.

4. The method for producing human A1 astrocytes according to any one of claims 1 to 3, wherein the step a includes:
a step a1 of differentiating human pluripotent stem cells or cells capable of differentiating into human astrocytes into human astrocytes other than human A1 astrocytes; and,
a step a2 of culturing the human astrocytes other than human A1 astrocytes, which are obtained in the step a1, in a presence of TNFα and IFNγ.

5. The method for producing human A1 astrocytes according to any one of claims 1 to 4, wherein the human astrocytes other than human A1 astrocytes, which are cultured in the step a, are human A2 astrocytes.

6. The method for producing human A1 astrocytes according to any one of claims 1 to 5, wherein the step a is culturing in a serum-free medium.

7. The method for producing human A1 astrocytes according to any one of claims 1 to 6, wherein in the step a, a concentration of TNFα in a medium is 0.01 ng/mL to 30 ng/mL.

8. The method for producing human A1 astrocytes according to any one of claims 1 to 7, wherein in the step a, a concentration of IFNγ in a medium is 0.1 ng/mL to 20 ng/mL.

9. The method for producing human A1 astrocytes according to any one of claims 1 to 8, wherein in the step a, a ratio of a concentration of TNFα in a medium to a concentration of IFNγ in the medium is 100:1 to 1:100.

10. An isolated human A1 astrocyte obtained by the method for producing human A1 astrocytes according to any one of claims 1 to 9.

11. A method for evaluating a test substance, comprising bringing a test substance into contact with the human A1 astrocytes according to claim 10.

12. The method for evaluating a test substance according to claim 11, further comprising evaluating a neuropathic change of the human A1 astrocytes after the bringing of the test substance into contact with the human A1 astrocytes according to claim 10.
